# EUROPÄISCHE PATENTANMELDUNG

(11) **EP 4 603 474 A1**
(43) Veröffentlichungstag der Anmeldung: **20.08.2025**
(21) Anmeldenummer: 24157567.9
(22) Anmeldetag: 14.02.2024
(51) Int. Cl.: C07C 67/31, C07C 69/24, C07C 69/675

(54) **VERFAHREN ZUR HERSTELLUNG EINES OMEGA-HYDROXYCARBONSÄUREESTERS AUS EINEM TRIOXANDERIVAT**

(71) Anmelder: Evonik Oxeno GmbH & Co. KG, 45772 Marl (DE)
(72) Erfinder: SCHAREINA, Thomas, 18195 Cammin (DE); SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Verfahren zur Herstellung eines omega-Hydroxycarbonsäureesters ausgehend von einem Trioxanderivaten.

## Beschreibung

Die vorliegende Erfindung betrifft ein Verfahren zur Herstellung eines omega-Hydroxycarbonsäureesters ausgehend von einem Trioxanderivaten.

Der vorliegenden Erfindung lag die Aufgabe zugrunde, ein Verfahren zur Herstellung von omega-Hydroxycarbonsäureestern bereitzustellen. Hierbei soll eine gute Ausbeute und eine gute Selektivität (n:iso) erzielt werden.

Diese Aufgabe wird gelöst durch ein Verfahren gemäß Anspruch 1.

Verfahren umfassend die Verfahrensschritte:
a) Vorlegen eines Trioxanderivats gemäß der Formel (**I**): 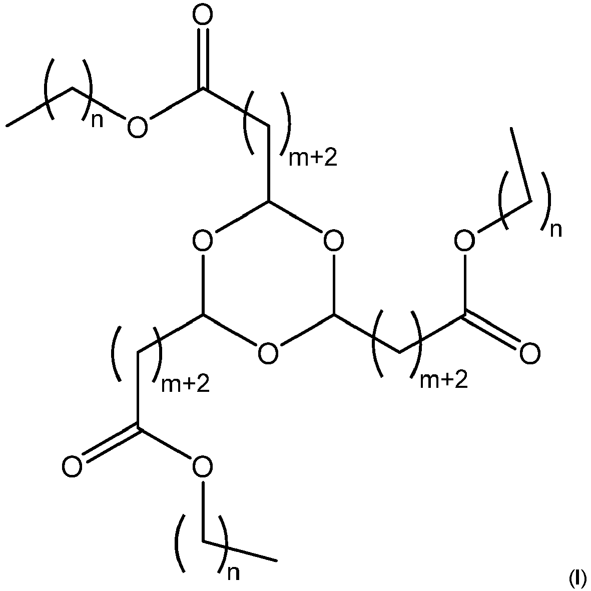
   wobei m für eine ganze Zahl von 1 bis 10 steht und
   n für eine ganze Zahl von 0 bis 8 steht;
b) Zugabe eines Phosphin-Liganden;
c) Zugabe einer Ru-Verbindung;
d) Zugabe von H₂O;
e) Zuführen von H₂;
f) Erwärmen des Reaktionsgemisches aus a) bis e), wobei das Trioxanderivat zu einem omega-Hydroxycarbonsäureester gemäß Formel (**II**) umgesetzt wird:

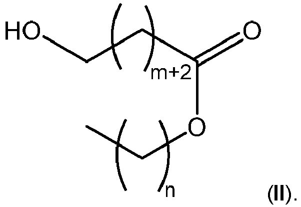

Hierbei können die Verfahrensschritte a) bis e) in beliebiger Reihenfolge erfolgen. Üblicherweise erfolgt die Zugabe von H₂ jedoch nachdem die Reaktionspartner in den Schritten a) bis d) vorgelegt wurden.

In einer Variante des Verfahrens steht m für eine ganze Zahl von 5 bis 9.

In einer Variante des Verfahrens steht m für 7.

In einer Variante des Verfahrens steht n für eine ganze Zahl von 0 bis 4 steht.

In einer Variante des Verfahrens steht n für 0.

In einer Variante des Verfahrens weist die Verbindung gemäß der Formel (**I**) die Struktur (**1**) auf:

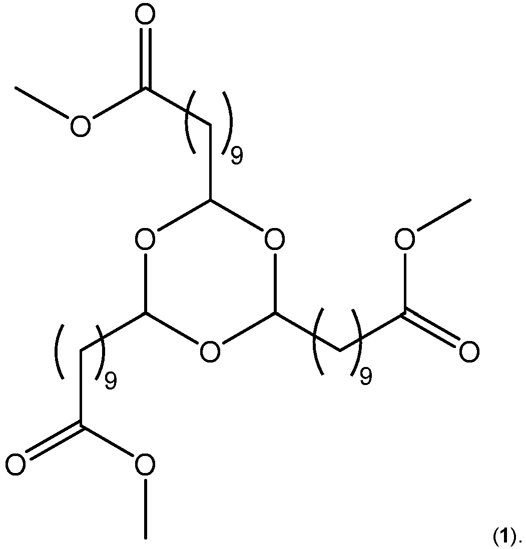

In einer Variante des Verfahrens weist der Phosphin-Liganden die Formel (**III**) auf: 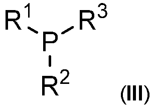 und wobei R¹, R², R³ ausgewählt sind aus: -(C₁-C₁₂)-Alkyl, -(C₆-C₂₀)-Aryl, -Cyclohexyl.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

In einer Variante des Verfahrens stehen R¹, R², R³ für -(C₆-C₂₀)-Aryl.

In einer Variante des Verfahrens ist der Phosphin-Ligand PPh₃.

In einer Variante des Verfahrens ist die Ru-Verbindung ausgewählt aus: RuCl₃ x 3H₂O, Ru₃(CO)₁₂, Ru(Cl)₂(DMSO)₄, Ru(acac)s.

In einer Variante des Verfahrens ist die Ru-Verbindung RuCl₃ x 3H₂O.

In einer Variante des Verfahrens weist das Verfahren eine zusätzlichen Verfahrensschritt d') auf: d') Zugabe eines organischen Lösungsmittels.

In einer Variante des Verfahrens ist das organischen Lösungsmittel ein Alkohol.

In einer Variante des Verfahrens erfolgt das Zuführen von H₂ bei einem Druck in einem Bereich von 1 MPa (10 bar) bis 5 MPa (50 bar).

In einer Variante des Verfahrens erfolgt das Zuführen von H₂ bei einem Druck in einem Bereich von 1 MPa (10 bar) bis 3 MPa (30 bar).

In einer Variante des Verfahrens erfolgt das Erwärmen in Verfahrensschritt f) auf eine Temperatur im Bereich von 40 °C bis 120 °C.

In einer Variante des Verfahrens erfolgt das Erwärmen in Verfahrensschritt f) auf eine Temperatur im Bereich von 60 °C bis 120 °C.

Im Folgenden soll die Erfindung anhand eines Ausführungsbeispiels näher erläutert werden.

### Synthese von Trimethyl-10, 10', 10"-(1,3,5-trioxan-2,4,6-triyl)tris(decanoat) (1)

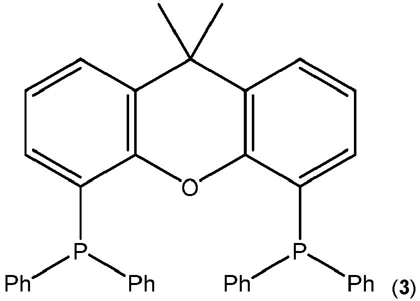

150 ml (132 g) Methyldec-9-enoate (**2**), 100 ml absolutes Toluol, 320 mg Ptl₂ (0,1 mol % bezüglich (**2**)), 620 mg Xantphos (**3**) (0,15 mol % bezüglich (**2**)) werden in einem 450 ml Hochdruckautoklaven (Parr Instruments) versehen mit Rührung und elektronischem Druckaufnehmer unter Argon platziert. Es werden 40 bar Synthesegas (H₂:CO = 1:1) aufgepresst und die Reaktion bei 60 °C unter Rührung (>500min⁻¹) durchgeführt. Die Reaktionszeit beträgt 10 h. Der Gasverbrauch wird nachreguliert, so dass die Reaktion bei ca. 40 bar stattfindet. Nach 10 h wird die Reaktion abgebrochen, der Autoklav heruntergekühlt, das Gas abgelassen und der Autoklav 4 mal mit 30 bar Stickstoff gespült. Die Reaktionslösung wird in einen 500 ml Schlenkkolben überführt.

Es wird ein GC gemacht. Die GC-Ausbeute an Methyl-11-oxoundecanoat (**4**) beträgt: 98 %, (Selektivität: n: iso = 98,2: 1,8).

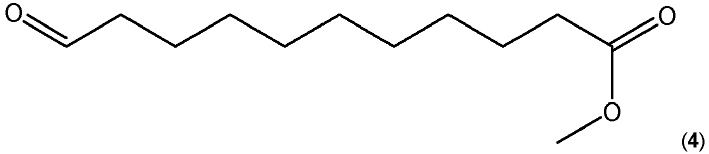

Anschliessend wird im Feinvakuum bei 10⁻³ Torr destilliert (K_{P} = 100 °C). Es resultiert eine farblose Flüssigkeit (146 g = 96 %).

Diese Flüssigkeit kristallisiert innerhalb von 24 h vollständig zu einem Feststoff durch, der als Trimethyl-10,10',10"-(1,3,5-trioxan-2,4,6-triyl)tris(decanoat) (**1**) mittels ¹H-, ¹³C-NMR- und MS -Analyse identifiziert wurde. Die Reinheit beträgt >99 %.

NMR (CDCl₃, 300 MHz):
¹H: 4,75 t(3 H, J_{HH} = 5,3 Hz), 3,59 s(9H), 2,23(6H, J_{HH} = 7,5 Hz), 1,6-1,5 m (12H) 1,38-1,16 m (36 H) ¹³C: 174,28 s ,101,65 s, 51,40 s, 34,39 s, 34,08 s, 29,38 s, 29,32 s, 29,20 s, 29,11 s, 24,93 s, 23,53 s
MS (70 ev, MZ (%)): 186(18), 171(44), 143(27), 139(65), 129(9), 121(17), 111(18), 98(36), 97(31), 87(78), 74(100), 69(43), 59(29), 57(17), 55(64).

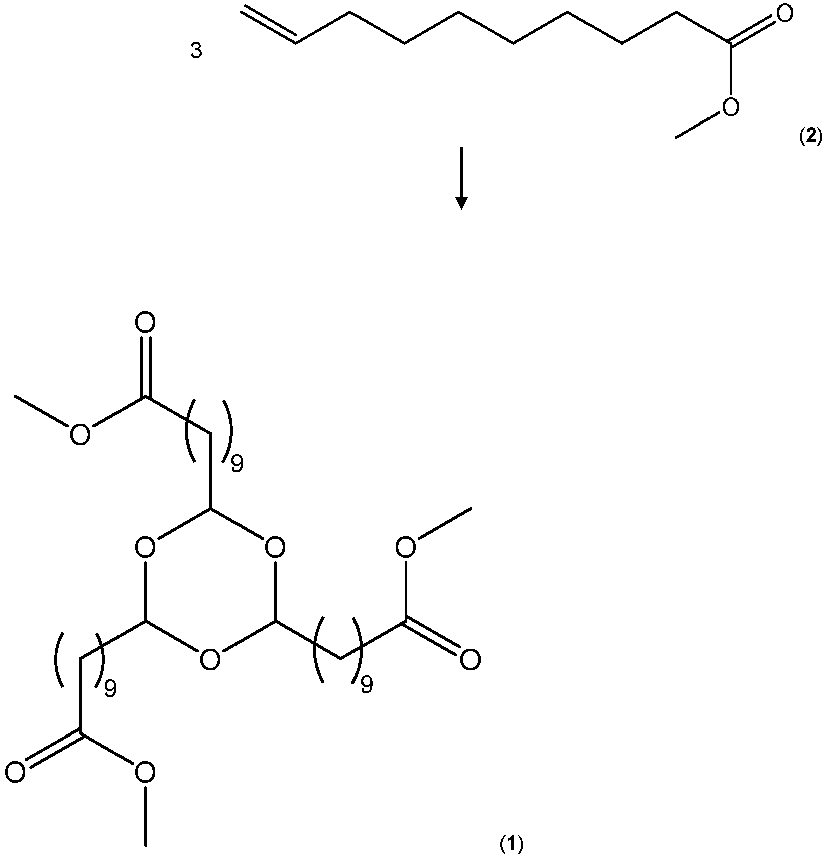

Reaktionsbedingungen:
Ester (**2**), 0,1 mol% Ptl₂, 0,15 mol% Xantphos (**3**), Lösungsmittel: Toluol, p(CO/H₂): 40 bar, T: 60 °C, t: 10 h.

### Synthese von Methyl-11-hydroxyundekanoat (5)

53,6 g (250 mmol) Trimethyl-10,10',10"-(1,3,5-trioxan-2,4,6-triyl)tris(decanoat) (**1**), 250 ml absolutes MeOH, 36 ml H₂O, 260 mg RuCl₃ x 3H₂O (0,4 mol % [Ru]), 1050 mg PPh₃ (1,6 mol %) werden in einem 450 ml Hochdruckautoklaven (Parr Instruments) versehen mit Rührung und elektronischem Druckaufnehmer unter Argon platziert. Es werden 20 bar Wasserstoff aufgepresst und die Reaktion bei 80 °C unter Rührung (>500min⁻¹) durchgeführt. Die Reaktionszeit beträgt 24 h. Der Gasverbrauch wird nachreguliert, so dass die Reaktion zwischen 20 bar und 15 bar stattfindet. Nach 24 h wird die Reaktion abgebrochen, der Autoklav heruntergekühlt, das Gas abgelassen. Der Autoklav wird 4 mal mit 30 bar Stickstoff gespült. Die Reaktionslösung wird in einen 500 ml Schlenkkolben überführt.

Es wird ein GC genommen. GC-Ausbeute an Methyl-11-oxoundecanoat beträgt > 99 %, (Selektivität = n:iso > 99:1).

Anschliessend wird im Feinvakuum bei 10⁻³ Torr destilliert (KP = 120 °C). Es resultiert eine farblose Flüssigkeit (50,2 g = 93%).

Diese Flüssigkeit kristallisiert innerhalb von 24 h zu einem Feststoff durch, der als Methyl-11-hydroxyundekanoat (**5**) mittels ¹H-NMR, ¹³C-NMR und MS-Analyse identifiziert wurde. Die Reinheit beträgt >99 %.

NMR (CDCl₃, 300 MHz): ¹H: 3,66 s(3 H), 3,63 t(2H, J_{HH} = 6,6 Hz), 2,3 t(2H, J_{HH} = 7,5 Hz), 1,7-1,5 m(4H ) 1,4-1,2 m(13H).

NMR (CDCl₃, 75 MHz): ¹³C: 174,38 s ,63,02 s, 51,46 s, 34,1 s, 32,77 s, 29,49 s, 29,37 s, 29,33 s, 29,21 s, 29,11 s, 25,91 s, 24,93 s.

Masse: 216,17

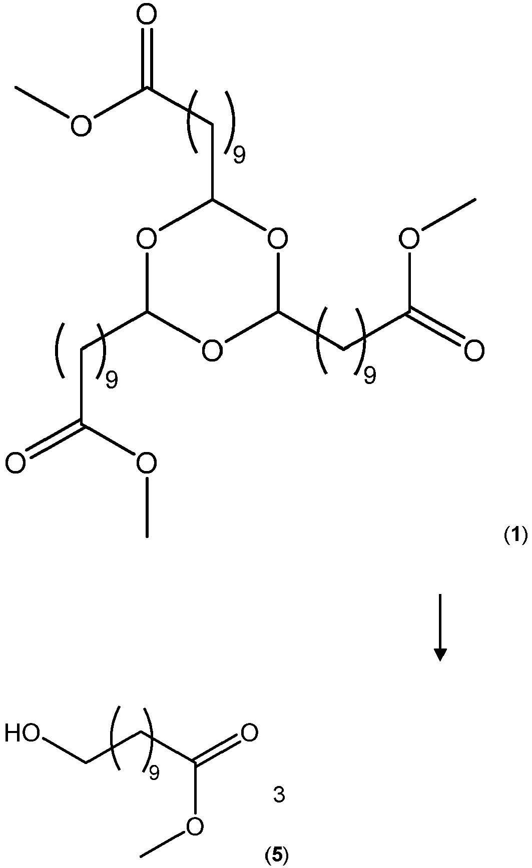

Reaktionsbedingungen:
Trioxanderivat (**1**), 0,4 mol% RuCl₃ x 3H₂O, 1,6 mol% PPh₃, Lösungsmittel: H₂O, MeOH, p(H₂): 20 bar, T: 80 °C, t: 24 h.

Wie die Versuchsergebnisse zeigen, wird die Aufgabe durch das erfindungsgemäße Verfahren gelöst.

## Patentansprüche

1. Verfahren umfassend die Verfahrensschritte:
a) Vorlegen eines Trioxanderivats gemäß der Formel (**I**): 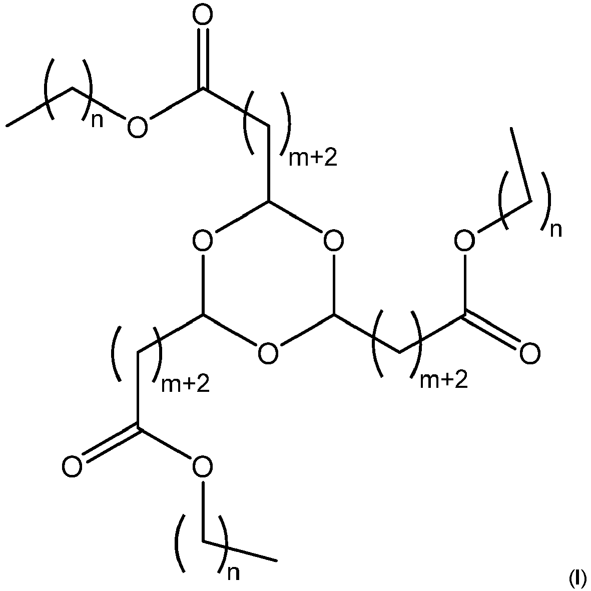
wobei m für eine ganze Zahl von 1 bis 10 steht und
n für eine ganze Zahl von 0 bis 8 steht;
b) Zugabe eines Phosphin-Liganden;
c) Zugabe einer Ru-Verbindung;
d) Zugabe von H₂O;
e) Zuführen von H₂;
f) Erwärmen des Reaktionsgemisches aus a) bis e), wobei das Trioxanderivat zu einem omega-Hydroxycarbonsäureester gemäß Formel (**II**) umgesetzt wird:

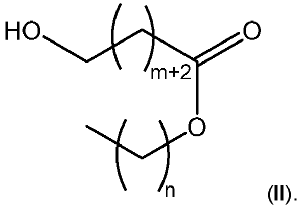

2. Verfahren nach Anspruch 1,
wobei m für eine ganze Zahl von 5 bis 9 steht.

3. Verfahren nach einem der Ansprüche 1 oder 2, wobei n für eine ganze Zahl von 0 bis 4 steht.

4. Verfahren nach einem der Ansprüche 1 bis 3,
wobei die Verbindung gemäß der Formel (**I**) die Struktur (**1**) aufweist:

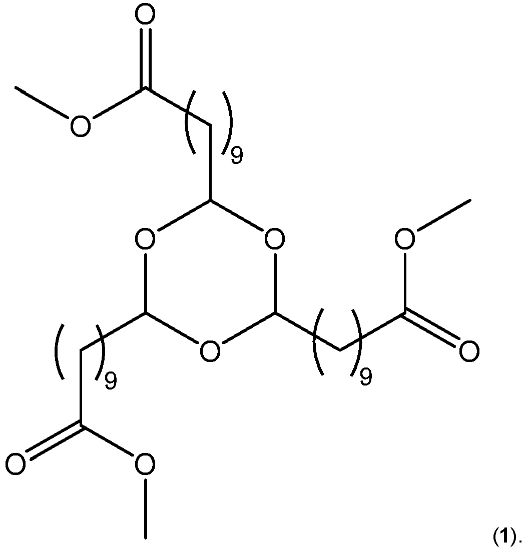

5. Verfahren nach einem der Ansprüche 1 bis 4,
wobei der Phosphin-Liganden die Formel (**III**) aufweist: 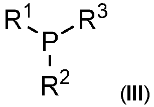
und wobei R¹, R², R³ ausgewählt sind aus: -(C₁-C₁₂)-Alkyl, -(C₆-C₂₀)-Aryl, -Cyclohexyl.

6. Verfahren nach Anspruch 5,
wobei R¹, R², R³ für -(C₆-C₂₀)-Aryl stehen.

7. Verfahren nach einem der Ansprüche 1 bis 6,
wobei der Phosphin-Ligand PPh₃ ist.

8. Verfahren nach einem der Ansprüche 1 bis 7,
wobei die Ru-Verbindung ausgewählt ist aus: RuCl₃ x 3H₂O, Ru₃(CO)₁₂, Ru(Cl)₂(DMSO)₄, Ru(acac)₃.

9. Verfahren nach einem der Ansprüche 1 bis 8,
wobei die Ru-Verbindung RuCl₃ x 3H₂O ist.

10. Verfahren nach einem der Ansprüche 1 bis 9,
wobei das Verfahren eine zusätzlichen Verfahrensschritt d') aufweist:
d') Zugabe eines organischen Lösungsmittels.

11. Verfahren nach Anspruch 10,
wobei das organische Lösungsmittel ein Alkohol ist.

12. Verfahren nach einem der Ansprüche 1 bis 11,
wobei das Zuführen von H₂ bei einem Druck erfolgt in einem Bereich von 1 MPa (10 bar) bis 5 MPa (50 bar).

13. Verfahren nach einem der Ansprüche 1 bis 12,
wobei das Erwärmen in Verfahrensschritt f) auf eine Temperatur im Bereich von 40 °C bis 120 °C erfolgt.
